# EUROPÄISCHE PATENTSCHRIFT

(11) **EP 0 804 601 B1**
(45) Veröffentlichungstag und Bekanntmachung des Hinweises auf die Patenterteilung: **14.03.2001**
(21) Anmeldenummer: 95931204.2
(22) Anmeldetag: 25.08.1995
(51) Int. Cl.: C12N 15/85, A61K 48/00, C12N 15/86

(54) **GENTHERAPEUTISCHE BEHANDLUNG VON TUMOREN MIT EINEM ENDOTHELZELLSPEZIFISCHEN, ZELLZYKLUSABHÄNGIGEN WIRKSTOFF**
GENETIC THERAPY OF TUMOURS WITH AN ENDOTHELIUM CELL-SPECIFIC ACTIVE SUBSTANCE WHICH IS DEPENDENT ON THE CELL CYCLE
TRAITEMENT DE TUMEURS PAR THERAPIE GENETIQUE AU MOYEN D'UNE SUBSTANCE ACTIVE SPECIFIQUE DE CELLULES ENDOTHELIALES ET DEPENDANT DU CYCLE CELLULAIRE

(30) Priorität: 26.08.1994 GB 9417366; 29.03.1995 GB 9506466
(43) Veröffentlichungstag der Anmeldung: 05.11.1997
(73) Patentinhaber: Aventis Pharma Deutschland GmbH, 65929 Frankfurt am Main (DE)
(72) Erfinder: SEDLACEK, Hans-Harald, D-35041 Marburg (DE); BOSSLET, Klaus, D-35037 Marburg (DE); MÜLLER, Rolf, D-35037 Marburg (DE)
(74) Vertreter: Dost, Wolfgang, Dr. rer.nat., Dipl.-Chem.
(86) Internationale Anmeldenummer: EP9503370
(87) Internationale Veröffentlichungsnummer: WO9606940

(56) Entgegenhaltungen:
- WO-A-93/10135
- WO-A-93/13807
- WO-A-94/29469

## Beschreibung

### Technisches Gebiet

Es wird eine DNA-Sequenz für die Gentherapie von Tumoren beschrieben. Die DNA-Sequenz besteht in ihren wesentlichen Elementen aus einer Aktivatorsequenz, einem Promotormodul und einem Gen für die Wirksubstanz.

Die Aktivatorsequenz wird zellspezifisch aktiviert in proliferierenden Endothelzellen oder diesen Endothelzellen benachbarte Zellen.

Diese Aktivierung wird zellzyklusspezifisch reguliert durch das Promotormodul.

Die Wirksubstanz stellt einen Inhibitor für die Angiogenese oder ein zytostatisches oder zytotoxisches Molekül dar. Die DNA-Sequenz wird eingefügt in einen viralen oder nicht-viralen Vektor, ergänzt um einen Liganden mit Affinität zur aktivierten Endothelzelle.

### 1. Tumorwachstum und Angiogenese

Die mangelnde Wirksamkeit vieler antitumoraler Wirkstoffe kann zumindest teilweise dadurch erklärt werden, daß die Tumorzellen innerhalb eines Tumorknotens für die antitumoralen, inbesondere hochmolekularen Wirkstoffe unerreichbar sind (Burrows et al., Pharm. Ther. 64, 155 (1994), Baxter et al., Microvasc. Rex. 41, 5 (1991)). Derartige Wirkstoffe müssen durch das Gefäßendothel, die Basalmembran und durch das Tumorstroma und -parenchym diffundieren, um jede einzelne Tumorzelle zu erreichen. Das Ausmaß dieser Diffusion wird im wesentlichen bestimmt durch die Konzentration bzw. das Konzentrationsgefälle des Wirkstoffes und seine physikochemischen Charakteristika. Des weiteren läuft der Diffusion die Konvektion zuwider, die vom höheren Druck im Tumorinneren nach auswärts gerichtet ist (Burrows et al., Microvasc. Res. 41, 5 (1991)).

Da Tumorgefäße auch für hochmolekulare Wirkstoffe erreichbar sind, wurde demzufolge schon früh vorgeschlagen (Denekamp, Prog. Appl. Microcirc. 4, 28 (1984), Denekamp, Cancer Topics 6, 6 1986), Denekamp, Cancer Metast. Rev. 9, 267 (1990), Denekamp, Brit. J. Radiol. 66, 181 (1993)), die Angiogenese, induziert durch Tumoren, für eine Tumortherapie zu nutzen.

So wurde versucht, über Substanzen, die die Angiogenese inhibieren, das Tumorwachstum zu hemmen (Bicknell et al., Semin. Cancer Biol. 3, 399 (1992)). Tierexperimentelle Untersuchungen zeigten, daß die systemische Verabreichung von Substanzen, welche die Angiogenese inhibieren, auch die Tumorproliferation inhibieren können. Dieses gilt beispielsweise für Suramin (Gagliardi et al., Cancer Rex. 52, 5073 (1992)), Heparin-Steroid-Konjugate (Thorpe et al., Cancer Res. 53, 3000 (1993)), O-(Chloroacetylcarbamoyl)fumigillol (Yamaoka et al., Cancer Res. 53, 4262 (1993)), für monoklonale Antikörper gegen Angiogenin (Olson et al., Cancer Res. 54, 4576 (1994)) und für Angiostatin (O'Reilly et al., Cell 79, 315 (1994)).

Nachteile der erwähnten Verfahren waren jedoch die systemische, nicht tumorspezifische Wirkung der Angiogeneseinhibitoren, ihre Nebenwirkungen und das Risiko eines erneuten Tumorwachstums nach Absetzen der Therapie.

Als Alternative wurde konzipiert, durch Schädigung von Endothelzellen die Blutversorgung der Tumoren derartig zu inhibieren, daß es zur Nekrose der Tumoren kommt (Denekamp, Brit. J. Radiol. 66, 181 (1993)). Im Sinne dieses Konzeptes wurde die Gabe von Toxinen, Zytostatika oder Isotopen, gekoppelt an Antikörper vorgeschlagen. Diese Antikörper sollten spezifisch sein für das Tumor-assoziierte Gefäßendothel. Die Antikörperkonjugate sollten das Tumor-assoziierte Gefäß endothelspezifisch zerstören und hierdurch eine Tumornekrose induzieren (Burrows et al., Pharma Ther. 64, 155 (1994)).

Ein weiterer Vorschlag war, an Tumor-assoziierte Endothelzellen über spezifische Antikörper thrombogene Substanzen, Cytokine oder Chemokine zu binden und über die hierdurch ausgelöste Gerinnung, Entzündung oder Immunregulation das Tumorwachstum zu beeinflussen. Ähnliche Effekte wurden mit dem Vorschlag angestrebt, über Endothelzell-spezifische Antikörper in Endothelzellen DNA einzuschleusen, welche die Endothelzellen transduzieren zur Sekretion von entzündlichen, immunregulatorischen oder das Wachstum von Tumorzellen beeinflussenden Substanzen (Burrows et al., Pharmac. Ther. 64, 155 (1994)).

Als Antigene für derartige Antikörper wurden Membranantigene auf der Oberfläche von Endothelzellen vorgeschlagen. Hierzu zählen beispielsweise Endoglin, Endosialin, p96 Dimer, VEGF-Rezeptoren, PDGF-Rezeptoren, Urokinase (uPA) Rezeptoren, und verschiedene Adhäsionsmoleküle (Burrows et al., Pharmac. Ther. 64, 155 (1994)).

All diesen Membranantigenen ist jedoch gemeinsam, daß sie auch auf nicht proliferierenden Endothelzellen, zumindest in geringeren Konzentrationen, vorkommen. Da jedoch nicht proliferierende Endothelzellen im Vergleich zu proliferierenden Endothelzellen auch in einem tumorbelasteten Organismus bei weitem überwiegen, ist hierdurch die notwendige Tumorspezifität der durch Gabe des Antikörperkonjugates angestrebten Wirksamkeit nicht in ausreichendem Maße gewährleistet.

### 2. Beschreibung des Wirkstoffes

Gegenstand der vorliegenden Erfindung ist nunmehr ein Wirkstoff, (d. h. ein Arzneimittel) welcher lokal wie auch systemisch Tumorpatienten gegeben werden kann und durch welchen am Ort des Tumorwachstums vorwiegend, wenn nicht ausschließlich in den proliferierenden Endothelzellen über einen längeren Zeitraum die Angiogenese gehemmt, eine Entzündung induziert oder zytostatische Substanzen direkt oder indirekt erzeugt werden, welche das Tumorwachstum inhibieren.

Zentraler Bestandteil dieses Wirkstoffes ist ein DNA-Konstrukt, welches aus folgenden Elementen besteht: 
Aktivatorsequenz
(UAS)
zellzyklus reguliertes
Promotormodul
antitumorale
Substanz
 (DNA wird im gesamten Text dieser Anmeldung als gemeinsamer Begriff sowohl für eine komplementäre (cDNA) wie auch für eine genomische DNA-Sequenz benutzt).

### 2.1. Wahl der Aktivatorsequenz

Als Aktivatorsequenz ist eine Nukleotidsequenz (Promotor- oder Enhancersequenz) zu verstehen, mit der Transkriptionsfaktoren, gebildet oder aktiv entweder in Endothelzellen oder aber in Zellen in unmittelbarer Nachbarschaft mit proliferierenden Endothelzellen, interagieren.

### a) Aktivatorsequenzen aktiviert in Endothelzellen

Als Aktivatorsequenz kann der CMV-Enhancer, der CMV-Promotor (EP 0173.177.B1), der SV40 Promotor oder jede andere, dem Fachmann bekannte Promotor- oder Enhancersequenz verwendet werden.

Im Sinne dieser Erfindung zählen zu den bevorzugten Aktivatorsequenzen jedoch solche genregulatorische Sequenzen bzw. Elemente aus Genen, die für besonders in Endothelzellen (oder aber in Zellen in unmittelbarer Nachbarschaft mit proliferierenden Endothelzellen) nachweisbare Proteine kodieren.

Einige dieser Proteine sind von Burrows et al. (Pharmac. Therp. 64, 155 (1994)) und Plate et al. (Brain Pathol. 4, 207 (1994)) beschrieben worden. Im besonderen zählen zu diesen Endothelzell-spezifischen Proteinen beispielsweise:
- Hirn-spezifischer, endothelialer Glucose-1 -Transporter
   Endothelzellen des Hirns zeichnen sich durch eine sehr starke Expression dieses Transporters aus, um den transendothelialen Transport von D-Glucose in das Hirn zu bewerkstelligen (Gerhart et al., J. Neurosci. Res. 22, 464 (1989)). Die Promotorsequenz wurde von Murakami et al. (J. Biol. Chem. 267, 9300 (1992)) beschrieben.
- Endoglin
   Endoglin scheint ein nicht signalübertragender Rezeptor des TGFβ zu sein (Gougos et al., J. Biol. Chem. 265, 8361 (1990), Cheifetz, J. Biol. Chem. 267, 19027 (1992), Moren et al., BBRC 189, 356 (1992)). In geringen Mengen kommt er auf normalem Endothel vor, ist jedoch verstärkt exprimiert auf proliferierendem Endothel (Westphal et al., J. Invest. Derm. 100, 27 (1993), Burrows et al., Pharmac. Ther. 64, 155 (1994)). Die Promotorsequenz wurde von Bellon et al. (Eur. J. Immunol. 23, 2340 (1993)) und Ge et al. (Gene 138, 201 (1994)) beschrieben.
- VEGF-Rezeptoren
   Zwei Rezeptoren werden unterschieden (Plate et al., Int. J. Cancer 59, 520 (1994)):
   * VEGF-Rezeptor-1 (flt-1)
      (de Vries et al., Science 255, 989 (1992))
      (enthält im zytoplasmischen Teil eine fms-ähnliche Tyrosinkinase) und der
   * VEGF-Rezeptor-2 (flk-1, KDR)
      (Terman et al., BBRC 187, 1579 (1992))
      (enthält im zytoplasmischen Teil eine Tyrosinkinase).

Beide Rezeptoren sind fast ausschließlich auf endothelialen Zellen (Senger et al., Cancer Metast. Rev. 12, 303 (1993)) anzutreffen.
- andere endothelspezifische Rezeptortyrosinkinasen
   * til-1 oder til-2
      (Partanen et al., Mol. Cell Biol. 12, 1698 (1992), Schnürch and Risau, Development 119, 957 (1993), Dumont et al., Oncogene 7, 1471 (1992))
   * B61-Rezeptor (Eck-Rezeptor)
      (Bartley et al., Nature 368, 558 (1994), Pandey et al., Science 268, 567 (1995), van der Geer et al., Ann. Rev. Cell Biol. 10, 251 (1994))
- B61
   Das B61-Molekül stellt den Liganden dar für den B61-Rezeptor. (Holzman et al., J. Am. Soc. Nephrol. 4, 466 (1993), Bartley et al., Nature 368, 558 (1994))
- Endothelin, im speziellen
   * Endothelin B
      (Oreilly et al., J. Cardiovasc. Pharm. 22, 18 (1993), Benatti et al., J. Clin. Invest. 91, 1149 (1993), O'Reilly et al., BBRC 193, 834 (1993). Die Promotorsequenz wurde von Benatti et al., J. Clin. Invest. 91, 1149 (1993) beschrieben.
   * Endothelin-1
      (Yanasigawa et al., Nature 332, 411 (1988).
      Die Promotorsequenz wurde von Wilson et al., Mol. Cell. Biol. 10, 4654 (1990) beschrieben.
- Endothelin-Rezeptoren, insbesondere der Endothelin-B-Rezeptor
   (Webb et al., Mol. Pharmacol. 47, 730 (1995), Haendler et al., J. Cardiovasc. Pharm. 20, 1 (1992)).
- Mannose-6-Phosphat-Rezeptoren
   (Perales et al., Eur. J. Biochem. 226, 225 (1994).
   Die Promotorsequenzen sind von Ludwig et al. (Gene 142, 311 (1994)), Oshima et al. (J. Biol. Chem. 263, 2553 (1988)) und Pohlmann et al. (PNAS USA 84, 5575 (1987)) beschrieben worden.
- von Willebrand Faktor
   Die Promotorsequenz wurde von Jahroudi und Lynch (Mol. Cell. Biol. 14, 999 (1994), Ferreira et al., Biochem. J. 293, 641 (1993) und Aird et al., PNAS USA 92, 4567 (1995)) beschrieben.
- IL-1α, IL-1β
   IL-1 wird von aktivierten Endothelzellen produziert (Warner et al., J. Immunol. 139, 1911 (1987)).
   Die Promotorsequenzen wurden von Hangen et al., Mol. Carcinog. 2, 68 (1986), Turner et al., J. Immunol. 143, 3556 (1989), Fenton et al., J. Immunol. 138, 3972 (1987), Bensi et al., Cell Growth Diff. 1, 491 (1990), Hiscott et al., Mol. Cell. Biol. 13, 6231 (1993) und Mori et al., Blood 84, 1688 (1994) beschrieben.
- IL-1-Rezeptor
   Die Promotorsequenz wurde von Ye et al., PNAS USA 90, 2295 (1993) beschrieben.
- Vascular Cell Adhesion Molecule (VCAM-1)
   Die Expression von VCAM-1 in Endothelzellen wird aktiviert durch Lipopolysaccharide, TNF-α (Neish et al., Mol. Cell. Biol. 15, 2558 (1995)), IL-4 (lademarco et al., J. Clin. Invest. 95, 264 (1995)), IL-1 (Marni et al., J. Clin. Invest. 92, 1866 (1993)).
   Die Promotorsequenz des VCAM-1 wurde beschrieben von Neish et al., Mol. Cell. Biol. 15, 2558 (1995), Ahmad et al., J. Biol. Chem. 270, 8976 (1995), Neish et al., J. Exp. Med. 176, 1583 (1992), lademarco et al., J. Biol. Chem. 267, 16323 (1992) und Cybulsky et al., PNAS USA 88, 7859 (1991).
- synthetische Aktivatorsequenz
   Als Alternative zu natürlichen endothelspezifischen Promotoren lassen sich auch synthetische Aktivatorsequenzen verwenden, die aus oligomerisierten Bindestellen für Transkriptionsfaktoren, die preferentiell oder selektiv in Endothelzellen aktiv sind, bestehen. Ein Beispiel hierfür ist der Transkriptionsfaktor GATA-2, dessen Bindestelle im Endothelin-1 Gen 5'-TTATCT-3' ist (Lee et al., Biol. Chem. 266, 16188 (1991), Dorfmann et al., J. Biol. Chem. 267, 1279 (1992) und Wilson et al., Mol. Cell. Biol. 10, 4854 (1990)).

### b) Aktivatorsequenzen, aktiviert in Zellen in Nachbarschaft aktivierter Endothelzellen

Bei proliferierenden Endothelien werden durch geöffnete "tight junctions" Nachbarzellen für Makromoleküle vom Blut aus zugänglich. Durch die funktionellen und anatomischen Wechselbeziehungen sind die Nachbarzellen von aktivierten Endothelzellen Zielzellen im Sinne dieser Erfindung.
- VEGF
   VEGF wird von unterschiedlichen Zellen (z.B. glatten Muskelzellen, Tumorzellen) in unmittelbarer Nachbarschaft zu proliferierenden Endothelzellen und besonders unter hypoxischen Bedingungen gebildet (Ferrara et al., Endoc. Rev. 13, 18 (1992), Berse et al., Mol. Biol. Cell 3, 211 (1992), Finkenzeller et al., BBRC 208, 432 (1995), Tischer et al., BBRC 165, 1198 (1989), Leung et al., Science 246, 1306 (1989)). Die genregulatorischen Sequenzen für das VEGF-Gen sind
   * die Promotorsequenz des VEGF-Gens (5' flankierende Region)
      (Michenko et al., Cell Mol Biol. Res. 40, 35 (1994), Tischer et al., J. Biol. Chem. 266, 11947 (1991)) oder
   * die Enhancersequenz des VEGF-Gens (3' flankierende Region)
      (Michenko et al., Cell Mol. Biol. Res. 40, 35 (1994) oder
   * das c-Src-Gen
      (Mukhopadhyay et al., Nature 375, 577 (1995), Bonham et al., Oncogene 8, 1973 (1993), Parker et al., Mol. Cell. Biol. 5, 831 (1985), Anderson et al., Mol. Cell. Biol. 5, 1122 (1985)) oder
   * das V-Src-Gen
      (Mukhodpadhyay et al., Nature 375, 577 (1995), Anderson et al., Mol. Cell. Biol. 5, 1122 (1985), Gibbs et al., J. Virol. 53, 19 (1985))
- Steroid-Hormonrezeptoren und deren Promotorelemente (Truss and Beato, Endocr. Rev. 14, 459 (1993)), insbesondere der
   * Maus-Mammatumor-Virus-Promotor
      In den meisten Zellen wird dieser Promotor durch Steroide aktiviert, so beispielsweise durch Glucocorticosteroide (Parks et al., Cell 8, 87 (1976)) oder durch Progestine (Cato et al., EMBO J. 5, 2237 (1986)).
      Die cDNA-Sequenz der Promotorregion der "long terminal repeat region" des MMTV wurde von Chalepakis et al., Cell 53, 371 (1988) und Truss und Beato (Endocr. Rev. 14, 459 (1993) beschrieben.

### 2.2. Wahl des Repressormoduls

Als zellzyklusreguliertes Promotormodul ist beispielsweise die Nukleotidsequenz CDE-CHR-Inr (siehe unten) zu verstehen. Die wesentliche Funktion dieses Promotormoduls ist die Hemmung der Funktion der Aktivatorsequenz in der G0/G1 Phase des Zellzyklus und eine Zellzyklus-spezifische Expression in der S/G2 Phase und damit in proliferierenden Zellen.

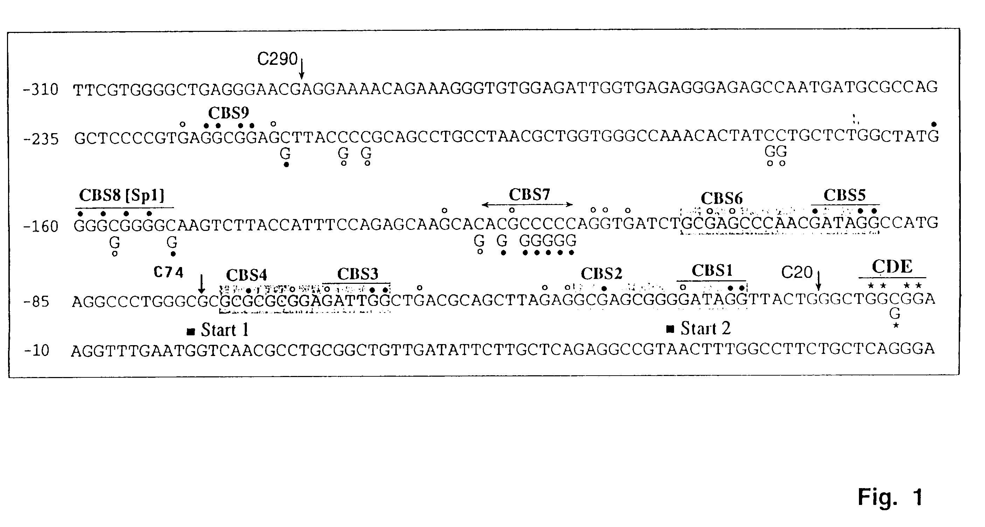

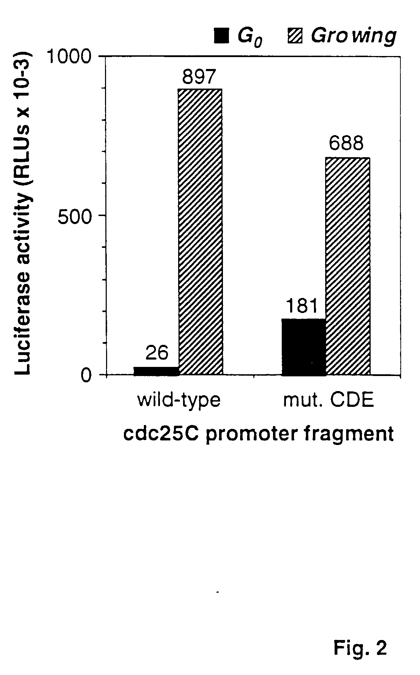

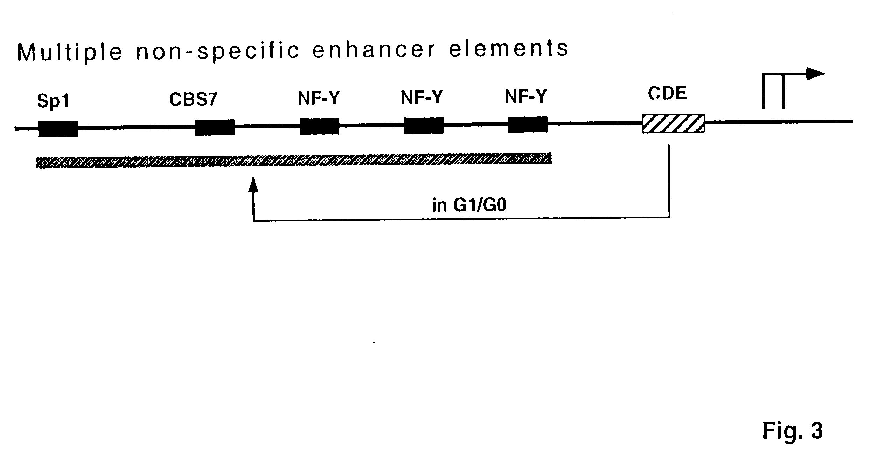

Das Promotormodul CDE-CHR-Inr wurde im Rahmen einer detaillierten Untersuchung der G2-spezifischen Expression des menschlichen cdc25C Promotors entdeckt. Ausgangspunkt war die Auffindung eines Repressorelementes ("cell cycle dependent element"; CDE), das für die Abschaltung des Promotors in der G1-Phase des Zellzyklus verantwortlich ist (Lucibello et al., EMBO J. 14, 132 (1995)). Durch genomisches Dimethylsulfat-(DMS)-Footprinting und funktionelle Analysen (Fig. 1, 2) konnte gezeigt werden, daß das CDE einen Repressor ("CDE-binding factor"; CDF) G1-spezifisch bindet und hierdurch zu einer Inhibition der Transkription in nichtproliferierenden (G0) Zellen führt. Das im Bereich des Basalpromotors lokalisierte CDE ist in seiner reprimierenden Funktion abhängig von einer "upstream activating sequence" (UAS). Dies führte zu dem Schluß, daß der CDE-bindende Faktor die transkriptionsaktivierende Wirkung 5' gebundener Aktivatorproteine in einer zellzyklusabhängigen Weise, d.h. in nichtproliferierenden Zellen sowie in der G1-Phase des Zellzyklus, hemmt (Fig. 3).

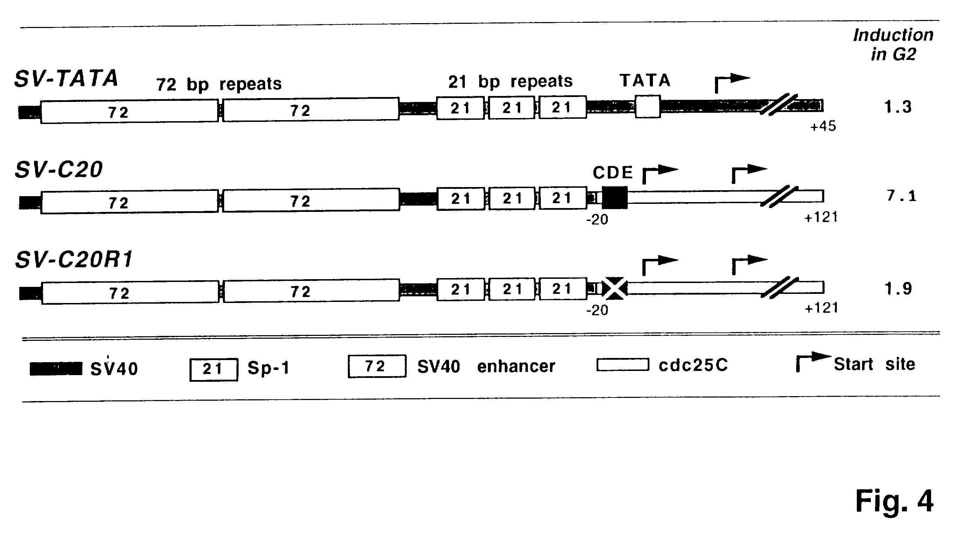

Diese Schlußfolgerung konnte durch ein weiteres Experiment bestätigt werden: Die Fusion des viralen, nicht-zellzyklusregulierten frühen SV40-Enhancers mit einem cdc25 Minimalpromotor (bestehend aus CDE und den 3' gelegenen Startstellen) führte zu einer klaren Zellzyklusregulation des chimären Promotors (Fig. 4). Nachfolgende Untersuchungen des cdc25C Enhancers haben gezeigt, daß es sich bei den vom CDF zellzyklusabhängig regulierten Transkriptionsfaktoren um NF-Y (Synonym: CBF; Dorn et al., Cell 50, 863 (1987), van Hujisduijnen et al., EMBO J. 9, 3119 (1990), Coustry et al., J. Biol. Chem. 270, 468 (1995)), Sp1 (Kadonaga et al., TIBS 11, 10, 1986)) und einen möglicherweise neuen an CBS7 bindenden Faktor (CIF) handelt. Ein weiterer interessanter Befund dieser Studie war die Beobachtung, daß NF-Y innerhalb des cdc25C Enhancers nur in Kooperation mit mindestens einem weiteren NF-Y Komplex oder mit CIF effizient die Transkription aktiviert. Sowohl NF-Y als auch Sp1 gehören zur Klasse der glutaminreichen Aktivatoren, was wichtige Hinweise auf den Mechanismus der Repression (z.B. Interaktion bzw. Interferenz mit bestimmten basalen Transkriptionsfaktoren oder TAFs) liefert.

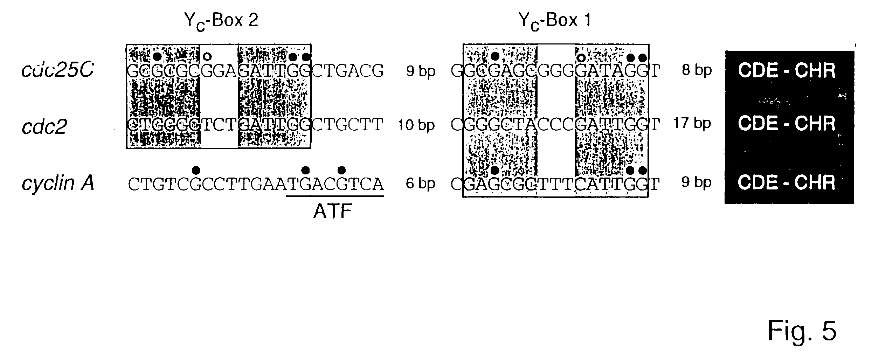

Ein Vergleich der Promotorsequenzen von cdc25C, cyclin A und cdc2 zeigte Homologien in mehreren Bereichen (Fig. 5). Nicht nur das CDE ist in allen 3 Promotoren konserviert (die vorhandenen Abweichungen sind funktionell nicht relevant), sondern auch die benachbarten Y_{C}-Boxen. Alle diese Bereiche zeigten wie erwartet Proteinbindung in vivo, im Falle des CDE in zellzyklusabhängiger Weise. Außerdem konnte gezeigt werden, daß alle 3 Promotoren durch eine Mutation des CDE dereguliert werden (Tabelle 1). Eine bemerkenswerte Ähnlichkeit wurde bei dem Vergleich der cdc25C, cyclin A und cdc2 Sequenzen auch im Bereich unmittelbar 3' vom CDE ("cell cycle genes homology region"; CHR) deutlich (Fig. 5). Dieser Bereich ist funktionell ebenso bedeutsam wie das CDE (Tabelle 1), wird in den in vivo DMS Footprinting Experimenten jedoch nicht sichtbar. Eine mögliche Erklärung hierfür ist eine Interaktion des Faktors mit der kleinen Furche der DNA. Ergebnisse aus "electrophoretic mobility shift assay" (EMSA) Experimenten deuten darauf hin, daß CDE und CHR gemeinsam einen Proteinkomplex, den CDF, binden. Diese Beobachtungen weisen daraufhin, daß die CDF-vermittelte Repression glutaminreicher Aktivatoren ein häufig vorkommender Mechanismus zellzyklusregulierter Transkription ist.

Von Bedeutung für die Regulation des cdc25C Promotors ist jedoch anscheinend nicht nur der CDE-CHR-Bereich, sondern auch eine der Initiationsstellen (Position +1) innerhalb der Nukleotidsequenz des basalen Promotors (Positionen ≤ -20 bis ≥ +30, siehe Fig. 1). Mutationen in diesem Bereich, welcher die in vitro Bindestelle für YY-1 (Seto und Shenk, Nature 354, 241 (1991), Usheva and Shenk, Cell 76, 1115 (1994)) einschließt, führen zu einer völligen Deregulation. In Anbetracht der Nähe des CDE-CHR zum basalen Promotor ist somit eine Interaktion des CDF mit dem basalen Transkriptionskomplex sehr wahrscheinlich.

### 2.3. Wahl der antitumoralen Substanz

### a) Inhibitoren der Proliferation

Als antitumorale Substanz im Sinne dieser Erfindung ist die DNA-Sequenz eines Proteins zu verstehen, welches die Proliferation von Endothelzellen inhibiert. Hierzu gehören beispielsweise die in folgenden Literaturzitaten aufgeführten DNA-Sequenzen für
- das Retinoblastomprotein (pRb/p110) oder für seine Analoga p107 und p120 (La Thangue, Curr. Opin. Cell Biol. 6, 443 (1994))
- das p53 Protein (Prives et al., Genes Dev. 7, 529 (1993))
- das p21 (WAF-1) Protein (EI-Deiry et al., Cell 75, 817 (1993))
- das p16 Protein (Serrano et al., Nature 366, 704 (1993), Kamb et al., Science 264, 436 (1994), Nobori et al., Nature 368, 753 (1994))
- weitere CdK-Inhibitoren (Übersicht bei Pines, TIBS 19, 143 (1995))
- das GADD45 Protein (Papathanasiou et al., Mol. Cell. Biol. 11, 1009 (1991), Smith et al., Science 266, 1376 (1994))
- das bak Protein (Farrow et al., Nature 374, 731 (1995), Chittenden et al., Nature 374, 733 (1995), Kiefer et al., Nature 374, 736 (1995))

Um eine schnelle intrazelluläre Inaktivierung dieser Zellzyklusinhibitoren zu verhindern, sind bevorzugt solche Gene zu verwenden, welche Mutationen für die Inaktivierungsstellen der exprimierten Proteine aufweisen, ohne daß diese hierdurch in ihrer Funktion beeinträchtigt werden.

Das Retinoblastomprotein (pRb) und die Verwandten p107 und p130 Proteine werden durch Phosphorylierung inaktiviert. Bevorzugt wird somit eine pRb/p110 -, p107 - oder p130 cDNA-Sequenz verwendet, die derartig punktmutiert ist, daß die Phosphorylierungsstellen des kodierten Proteins gegen nicht phosphorylierbare Aminosäuren ausgetauscht sind.

Entsprechend Hamel et al. (Mol. Cell Biol. 12, 3431 (1992)) wird das Retinoblastomprotein (pRb/p110) durch Austausch der Aminosäuren in den Positionen 246, 350, 601, 605, 780, 786, 787, 800 und 804 nicht mehr phosphorylierbar, seine Bindungsaktivität mit dem großen T-Antigen wird jedoch nicht beeinträchtigt. Beispielsweise werden beim pRb/p110 Protein die Aminosäuren Thr-256, Ser-601, Ser-605, Ser-780, Ser-786, Ser-787 und Ser-800 mit Ala, die Aminosäure Thr-350 mit Arg und die Aminosäure Ser-804 mit Glu ausgetauscht.

In analoger Weise wird die DNA-Sequenz für das p107 - oder p130 Protein mutiert.

Das Protein p53 wird in der Zelle inaktiviert entweder durch Bindung an spezielle Proteine, wie beispielsweise MDM2 oder durch Oligomerisierung des p53 über das dephosphorylierte C-terminale Serin 392 (Schikawa et al., Leukemia und Lymphoma 11, 21 (1993) und Brown, Annals of Oncology 4, 623 (1993)). Bevorzugt wird somit eine p53 cDNA-Sequenz verwendet, welche C-Terminal verkürzt ist um das Serin 392.

### b) Angiogeneseinhibitoren

Als antitumorale Substanz ist des weiteren die DNA-Sequenz für ein Protein zu verstehen, welches die Angiogenese inhibiert. Zu diesen Proteinen zählt insbesondere (in der aufgeführten Literatur wird Bezug genommen zur jeweiligen DNA-Sequenz) beispielsweise:
- Plasminogenaktivatorinhibitor-1 (PAI-1)
   (Reilly et al., J. Biol. Chem. 265, 9570 (1990), Bosma et al., J. Biol. Chem. 253, 9219 (1988))
- PAI-2
   (Steven et al., Eur. J. Biochem. 196, 431 (1991))
- PAI-3
   (Meijers et al., J. Biol. Chem. 266, 15028 (1991))
- Angiostatin
   (O'Reilly et al., Cell 79, 315 (1994), Bicknell et al., Semin. Cancer Biol. 3, 399 (1992))
- Interferone
   (Dorr, Drugs 45, 177 (1993), Drugs of Today 22, 597 (1986), US 45 185 84, US 45 88 585), im speziellen
   * IFNα
      (Henco et al., J. Mol. Biol. 185, 227 (1985), Pestka et al., Ann. Rev. Biochem. 56, 727 (1987), Weissmann et al., Phil. Trans. R. Soc. Lond. B299, 7 (1982), Goeddel et al., Nature 290, 20 (1981))
   * IFNβ
      (Sen et al., J. Biol. Chem. 267, 5017 (1992), Mark et al., EP 192 811, EP 234 599, US 45 88 585)
   * IFNγ
      (Gray et al., Nature 295, 503 (1982), Yip et al., PNAS USA 79, 1820 (1982), Rinderknecht et al., J. Biol. Chem. 259, 6790 (1984))
- Platelet factor 4
   (Mc Manus et al., J. Immunol. 123, 2835 (1979); Brindley et al., J. Clin.
   Invest. 72, 1218 (1983); Deuel et al., Proc. Natl. Acad. Sci. USA 78, 4584, 1981))
- IL-12
   (Voest et al., N. Natl. Cancer Inst. 87, 581 (1995), Wolf et al., J. Immunol. 146, 3074 (1991), Gubler et al., PNAS USA 88, 4143 (1991), Kobayashi et al., J. Exp. Med. 170, 827 (1989), Gabler et al., PNAS 88, 4143 (1991), Gately et al., J. Immunol. 147, 874 (1991), Schoenhaut et al., J. Immunol. 148, 3433 (1992), Wolf et al., J. Immunol. 146, 3074 (1991))
- TIMP-1
   (Kolkenbrock et al., Eur. J. Biochem. 198, 775 (1991), Faucher et al., Path. Biol. 37, 199 (1989))
- TIMP-2
   (Kolkenbrock et al., Eur. J. Biochem. 198, 775 (1991))
- TIMP-3
   (Wick et al., J. Biol. Chem. 269, 18953 (1994)).
- Leukemia Inhibitory Factor (LIF)
   (Pepper et al., J. Cell Science 108, 73 (1995), Gough et al., Ciba Found. Symp. 167, 24 (1992), PNAS USA 85, 5971 (1988), Stahl et al., J. Biol. Chem. 265, 8833 (1990), Rathjan et al., Cell 62, 1105 (1990))

### c) zytostatische und zytotoxische Proteine

Als antitumorale Substanz ist jedoch auch eine DNA-Sequenz für ein Protein zu verstehen, welches direkt oder indirekt eine zytostatische Wirkung auf Tumoren aufweisen. Hierzu zählen im besonderen
- Perforin
   (Lin et al., Immunol. Today 16, 194 (1995))
- Granzym
   (Smyth et al., Immunol. Today 16, 202 (1995))
- IL-2
   (Fletscher et al., Lymphok. Res. 6, 45 (1987), Matsui et al., Lymphokines 12, 1 (1985), Tanaguchi et al., Nature 302, 305 (1983))
- IL-4
   (Lee et al., PNAS 83, 2061 (1986); Paul, Blood 77, 1859 (1991), Yokota et al., PNAS USA 83, 5894 (1986), van Leuven et al., Blood 73, 1142 (1989), Arai et al., J. Immunol. 42, 274 (1989)
- IL-12
   (Kobayashi et al., J. Exp. Med. 170, 827 (1989), Gabler et al., PNAS 88, 4143 (1991), Gately et al., J. Immunol. 147, 874 (1991), Schoenhaut et al., J. Immunol. 148, 3433 (1992), Wolf et al., J. Immunol. 146, 3074 (1991))
- Interferone, wie beispielsweise
   * IFN-α
      (Henco et al., J. Mol. Biol. 185, 227 (1985), Pestka et al., Ann. Rev.
      Biochem. 56, 727 (1987), Weissmann et al., Phil. Trans. R. Soc. Lond. B299, 7 (1982), Goeddel et al., Nature 290, 20 (1981))
   * IFNβ
      (Sen et al., J. Biol. Chem. 267, 5017 (1992), Mark et al., EP192 811, EP 234 599, US 45 88 585)
   * IFNγ
      (Gray et al., Nature 295, 503 (1982), Yip et al., PNAS USA 79, 1820 (1982), Rinderknecht et al., J. Biol. Chem. 259, 6790 (1984))
- TNF
   (Porter, TiBTech 9, 158 (1991); Sidhu et al., Pharmac. Ther. 57, 79 (1993)), im speziellen
   * TNFα
      (Beutler et al., Nature 320, 584 (1986), Kriegler et al., Cell 53, 45 (1988))
   * TNFβ
      (Gray et al., Nature 312, 721 (1984), Li et al., J. Immunol. 138, 4496 (1987), Aggarwal et al., J. Biol. Chem. 260, 2334 (1985))
- Oncostatin M
   (Brown et al., J. Immunol. 147, 2175 (1991); Grove et al., J. Biol. Chem. 266, 18194 (1991); Hamilton et al., Biochem. Biophys. Res. Commun. 180, 652 (1991), Malik et al., Mol. Cell. Biol. 9, 2847 (1989), Kallstad et al., J. Biol. Chem. 266, 8940 (1991))

### d) Induktoren von Entzündungen

Als antitumorale Substanz ist des weiteren die DNA-Sequenz für ein Protein zu verstehen, welches ggf. zusätzlich zur antitumoralen Wirkung Entzündungen stimulieren und hierdurch zur Elimination von Tumorzellen beiträgt. Hierzu zählen im besonderen beispielsweise:
- RANTES (MCP-2)
   (Schall et al., J. Immunol. 141, 1018 (1988), Cell 61, 361 (1990))
- monocyte chemotactic and activating factor (MCAF)
   (Zachariae et al., J. Exp. Med. 171, 2177 (1990); Rollins et al., Blood 78, 1112 (1991); Mukaida et al., J. Immunol. 146, 1212 (1991); Sica et al., J. Immunol. 144, 3034 (1990))
- IL-8
   (Lotz et al., J. Immunol. 148, 466 (1992); Clore et al., Biochemistry 29, 1689 (1990), Matsushima et al., J. Exp. Med. 167, 1883 (1988), Baglioni et al., J. Clin. Invest. 84, 1045 (1989), Int. J Immunopharm. 17, 103 (1995), Carre et al., J. Clin. Invest. 88, 1802 (1991))
- macrophage inflammatory protein-1 (MIP-1α, -β)
   (Broxmeyer et al., J. Immunol. 147, 2586 (1991); Oh et al., J. Immunol. 147, 2978 (1991); Graham et al., Nature 344, 442 (1990))
- neutrophil activating protein-2 (NAP-2)
   (Walz, Rheum. Arthr., London (1991), Chang et al., J. Biol. Chem. 269, 25277 (1994))
- IL-3
   (Otsuka et al., J. Immunol. 140, 2288 (1988); de Vries et al., Stem Cells 11, 72 (1993), Yang et al., Blood 71, 958 (1988), Cell 47, 3 (1986), Philips et al., Gene 84, 501 (1989))
- IL-5
   (Azuma et al., Nucl. Acid Res. 14, 9149 (1986); Yokota et al., PNAS 84, 7388 (1987); Campbell et al., PNAS 84, 6629 (1987), Azuma et al., Nucl. Acids Res. 14, 9149 (1986))
- human leukemia inhibitory factor (LIF)
   (Gough et al., Ciba Found. Symp. 167, 24 (1992), PNAS USA 85, 5971 (1988), Stahl et al., J. Biol. Chem. 265, 8833 (1990), Rahtjan et al., Cell 62, 1105(1990))
- IL-7
   (Matzuda et al., Leuk. Lymph. 5, 231 (1991), Lupton et al., J. Immunol. 144, 3592 (1990), Goodwin et al., PNAS USA 86, 302 (1989), Swatherland et al., Hum. Genet. 82, 371 (1989))
- IL-11
   (Paul et al., Proc. Natl. Acad. Sci. USA 87, 7512 (1990), Teramura et al.,
   Blood 79, 327 (1992), Kawashima et al., FEBS Lett. 283, 199 (1991))
- IL-13
   (McKenzie et al., J. Immunol. 150, 5436 (1993), Muzio et al., Blood 83, 1738 (1994), McKenzie et al., PNAS 90, 3735 (1993), Minty et al., Nature 362, 248 (1993))
- GM-CSF
   (Wong et al., Science 228, 810 (1985), Gough et al., Nature 309, 763 (1984), Nicola et al., J. Biol. Chem. 254, 5290 (1979))
- G-CSF
   (Nagata et al., EMBO J. 5, 575 (1986), Nagata et al., Nature 319, 415 (1986), Souza et al., Science 232, 61 (1986))
- M-CSF
   (Welte et al., PNAS 82, 1526 (1985), Lu et al., Arch. Biochem. Biophys. 268, 81 (1989), Kawasaki et al., Science 230, 291 (1985), Suzu et al., J. Biol. Chem. 267, 4345 (1992), Wong et al., Science 235, 1504 (1987))

Als Wirksubstanz in Sinne der Erfindung können auch DNA-Sequenzen von Fusionsproteinen zwischen den aufgeführten Cytokinen zum einen und dem Fc-Teil des menschlichen Immunglobulins zum anderen Verwendung finden. Derartige DNA-Sequenzen und ihre Herstellung wurden in der EPA 0464 633 A1 beschrieben.

### e) Enzyme für die Aktivierung von Vorstufen von Zytostatika

Als antitumorale Substanz ist jedoch auch die DNA-Sequenz für ein Enzym zu verstehen, welches in der Lage ist, Vorstufen eines antitumoralen Wirkstoffes in einen antitumoralen Wirkstoff zu überführen.

Derartige Enzyme, welche inaktive Vorsubstanzen (Prodrugs) in aktive Zytostatika (Drugs) spalten und die jeweils zugehörigen Prodrugs und Drugs sind bereits von Deonarain et al. (Br. J. Cancer 70, 786 (1994), von Mullen, Pharmac. Ther. 63, 199 (1994) und Harris et al., Gene Ther. 1, 170 (1994)) übersichtlich beschrieben worden.

Beispielsweise ist die DNA-Sequenz eines der folgenden Enzyme zu verwenden:
- Herpes Simplex Virus Thymidinkinase
   (Garapin et al., PNAS USA 76, 3755 (1979), Vile et al., Cancer Res. 53, 3860 (1993), Wagner et al., PNAS USA 78, 1441 (1981), Moelten et al., Cancer Res. 46, 5276 (1986), J. Natl. Cancer Inst. 82, 297 (1990))
- Varizella Zoster Virus Thymidinkinase
   (Huber et al., PNAS USA 88, 8039 (1991), Snoeck, Int. J. Antimicrob. Agents 4, 211 (1994))
- bakterielle Nitroreduktase
   (Michael et al., FEMS Microbiol. Letters 124, 195 (1994), Bryant et al., J. Biol. Chem. 266, 4126 (1991), Watanabe et al., Nucleic Acids Res. 18, 1059 (1990))
- bakterielle β-Glucuronidase
   (Jefferson et al., PNAS USA 83, 8447 (1986)
- pflanzliche β-Glucuroniase aus Secale cereale
   (Schulz et al., Phytochemistry 26, 933 (1987))
- humane β-Glucuronidase
   (Bosslet et al., Br. J. Cancer 65, 234 (1992), Oshima et al., PNAS USA 84, 685 (1987))
- humane Carboxypeptidase (CB) z.B.
   * CB-A der Mastzelle
      (Reynolds et al., J. Clin. Invest. 89, 273 (1992))
   * CB-B des Pankreas
      (Yamamoto et al., J. Biol. Chem. 267, 2575 (1992), Catasus et al., J. Biol. Chem. 270, 6651 (1995))
   * bakterielle Carboxypeptidase
      (Hamilton et al., J. Bacteriol. 174, 1626 (1992), Osterman et al., J. Protein Chem. 11, 561 (1992))
- bakterielle β-Laktamase
   (Rodrigues et al., Cancer Res. 55, 63 (1995), Hussain et al., J. Bacteriol. 164, 223 (1985), Coque et al., Embo J. 12, 631 (1993)
- bakterielle Cytosindeaminase
   (Mullen et al., PNAS USA 89, 33 (1992), Austin et al., Mol. Pharmac. 43, 380 (1993), Danielson et al., Mol. Microbiol. 6, 1335 (1992)
- humane Catalase bzw. Peroxidase
   (Ezurum et al., Nucl. Acids Res. 21, 1607 (1993))
- Phosphatase, im besonderen
   * humane alkalische Phosphatase
      (Gum et al., Cancer Res. 50, 1085 (1990))
   * humane saure Prostataphosphatase
      (Sharieff et al., Am. J. Hum. Gen. 49, 412 (1991), Song et al., Gene 129, 291 (1993), Tailor et al., Nucl. Acids Res. 18, 4928 (1990))
   * Typ 5 saure Phosphatase
      (Gene 130, 201 (1993))
- Oxidase, im besonderen
   * humane Lysyloxidase
      (Kimi et al., J. Biol. Chem. 270, 7176 (1995))
   * humane saure D-aminooxidase
      (Fukui et al., J. Biol. Chem. 267, 18631 (1992))
- Peroxidase, im besonderen
   * humane Gluthation Peroxidase
      (Chada et al., Genomics 6, 268 (1990), Ishida et al., Nucl. Acids Res. 15, 10051 (1987))
   * humane Eosinophilen Peroxidase
      (Ten et al., J. Exp. Med. 169, 1757 (1989), Sahamaki et al., J. Biol. Chem. 264, 16828 (1989))
   * humane Schilddrüsen Peroxidase
      (Kimura, PNAS USA 84, 5555 (1987)).

Zur Erleichterung der Sekretion der aufgeführten Enzyme kann die jeweils in der DNA-Sequenz enthaltende homologe Signalsequenz ersetzt werden durch eine heterologe, die extrazelluläre Ausschleusung verbessernde Signalsequenz.

So kann beispielsweise die Signalsequenz der β-Glucuronidase (DNA Position≤ 27 bis 93; Oshima et al., PNAS 84, 685 (1987)) ersetzt werden durch die Signalsequenz für das Immunglobulin (DNA Position ≤ 63 bis ≥ 107; Riechmann et al., Nature 332, 323 (1988)).

Desweiteren sind bevorzugt DNAs solcher Enzyme zu wählen, welche durch Punktmutationen in einem geringeren Maße in Lysosomen gespeichert und vermehrt sekretiert werden. Derartige Punktmutationen wurden beispielsweise für die β-Glucuronidase beschrieben (Shiplex et al., J. Biol. Chem. 268, 12193 (1993)).

### 2.4. Kombination von mehreren antitumoralen Substanzen

Gegenstand der Erfindung ist des weiteren ein Wirkstoff, in welchem eine Kombination der DNA-Sequenzen von mehreren gleichen antitumoralen Substanzen (A,A) oder von unterschiedlichen antitumoralen Substanzen (A,B) vorliegt. Zur Expression von zwei DNA-Sequenzen wird vorzugsweise die cDNA einer "internal ribosome entry site" (IRES) als regulatorisches Element zwischengeschaltet.

Aktivator-
sequenz
(UAS)

zellzyklus-
reguliertes
Promotor-
modul

antitumorale
Substanz
A

internal
ribosome
entry
site

antitumorale
Substanz
A oder B

Derartige IRES wurden beispielsweise von Mountford und Smith (TIG 11, 179 (1995), Kaufman et al., Nucl. Acids Res. 19, 4485 (1991), Morgan et al., Nucl. Acids Res. 20, 1293 (1992, Dirks et al., Gene 128, 247 (1993), Pelletier und Sonenberg, Nature 334, 320 (1988) und Sugitomo et al., BioTechn. 12, 694 (1994) beschrieben.

So kann die cDNA der IRES-Sequenz des Poliovirus (Position ≤ 140 bis ≥ 630 des 5' UTR; Pelletier und Sonenberg, Nature 334, 320 (1988)) zur Verknüpfung der DNA der antientzündlichen Substanz A (am 3' Ende) und der DNA der antientzündlichen Substanz B (am 5'Terminus) verwendet werden.

Ein derartiger Wirkstoff weist je nach Kombination additive (A+A, A+B1) oder synergistische Wirkung im Sinne der Erfindung auf.

### 2.5. Konstruktion des Vektors

Das erfindungsgemäße DNA-Konstrukt wird in einer dem Fachmann geläufigen Weise zu einem Vektor vervollständigt. Dieser Vektor kann viralen oder nicht-viralen Ursprungs sein. Beispielsweise wird das erfindungsgemäße DNA-Konstrukt in einen viralen Vektor eingefügt (siehe hierzu D. Jolly, Cancer Gene Therapy 1, 51 (1994)), oder aber als Plasmid verwendet. Virale Vektoren oder Plasmide können mit kolloidalen Dispersionen komplexiert werden, so beispielsweise mit Liposomen (Farhood et al., Annals of the New York Academy of Sciences 716, 23 (1994)) oder aber mit einem Polylysin-Ligand-Konjugaten (Curiel et al., Annals of the New York Academy of Sciences 716, 36 (1994)) oder sonstigen gebräuchlichen Hilfsstoffen als Arzneimittel konfektioniert werden.

### 2.6. Auswahl des Liganden

Virale und nicht-virale Vektoren können mit einem Liganden ergänzt werden. Als Liganden, beispielsweise in Polylysin-Ligand-Konjugaten, werden Substanzen bevorzugt, welche an die Oberfläche von proliferierenden Endothelzellen binden. Hierzu gehören Antikörper oder Antikörperfragmente, gerichtet gegen Membranstrukturen von Endothelzellen wie sie beispielsweise von Burrows et al. (Pharmac. Ther. 64, 155 (1994)), Hughes et al. (Cancer Res. 49, 6214 (1989) und Maruyama et al. (PNAS-USA 87, 5744 (1990) beschrieben wurden. Insbesondere zählen hierzu Antikörper gegen die VEGF-Rezeptoren.

Die murinen monoklonalen Antikörper sind bevorzugt in humanisierter Form einzusetzen. Die Humanisierung erfolgt in der von Winter et al. (Nature 349, 293 (1991) und Hoogenbooms et al. (Rev. Tr. Transfus. Hemobiol. 36, 19 (1993)) dargestellten Weise. Antikörperfragmente werden entsprechend dem Stand der Technik hergestellt, beispielsweise in der von Winter et al., Nature 349, 293 (1991), Hoogenboom et al., Rev. Tr. Transfus. Hemobiol. 36, 19 (1993), Girol, Mol. Immunol. 28, 1379 (1991) oder Huston et al., Intern. Rev. Immunol. 10, 195 (1993) beschriebenen Weise.

Zu den Liganden gehören des weiteren alle Wirkstoffe, welche an Membranstrukturen oder Membranrezeptoren auf Endothelzellen binden. Beispielsweise gehören hierzu Substanzen, die endständig Mannose enthalten des weiteren IL-1 oder Wachstumsfaktoren oder deren Fragmente bzw. Teilsequenzen von ihnen, die an Rezeptoren exprimiert durch Endothelzellen binden, wie beispielweise PDGF, bFGF, VEGF, TGFβ (Pusztain et al., J. Pathol. 169, 191 (1993)). Desweiteren gehören hierzu Adhäsionsmoleküle, welche an aktivierte und/oder proliferierende Endothelzellen binden. Derartige Adhäsionsmoleküle, wie beispielsweise SLex, LFA-1, MAC-1, LECAM-1 oder VLA-4, wurden bereits beschrieben (Übersichten bei Augustin-Voss et al., J. Cell Biol. 119, 483 (1992), Pauli et al., Cancer Metast. Rev. 9, 175 (1990), Honn et al., Cancer Metast. Rev. 11, 353 (1992)).

### 2.7. Herstellung des Wirkstoffes (Beispiele)

Die Herstellung des erfindungsgemäßen Wirkstoffes wird an Hand folgender Beispiele näher beschrieben:

### a) Konstruktion des chimären Promotors Endothelin 1-CDE-CHR-Inr

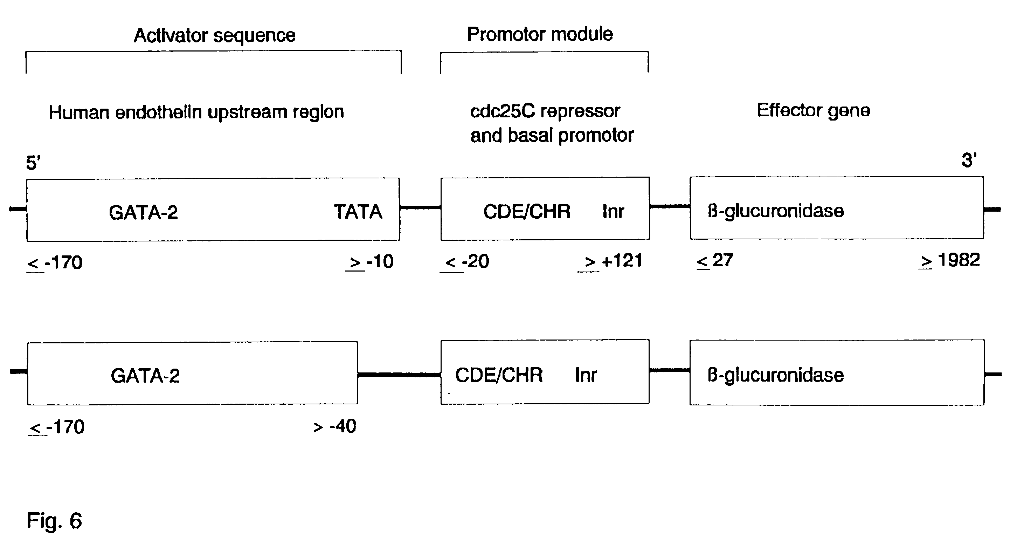

Der humane Endothelin-1 Promotor (Position ≤ -170 bis ≥ -10) oder eine um die TATA-Box verkürzte Variante (Position ≤ -170 bis ≥ -40) werden an ihrem 3' Ende mit dem 5'-Terminus des CDE-CHR-Inr Moduls (Position ≤ -20 bis ≥ +121) des humanen cdc25C-Gens verknüpft (Fig. 6). Die Verknüpfung erfolgt mit Hilfe von dem Fachmann bekannten und käuflichen Enzymen.

### b) Konstruktion eines Plasmids enthaltend den zentralen Bestahdteil des Wirkstoffes

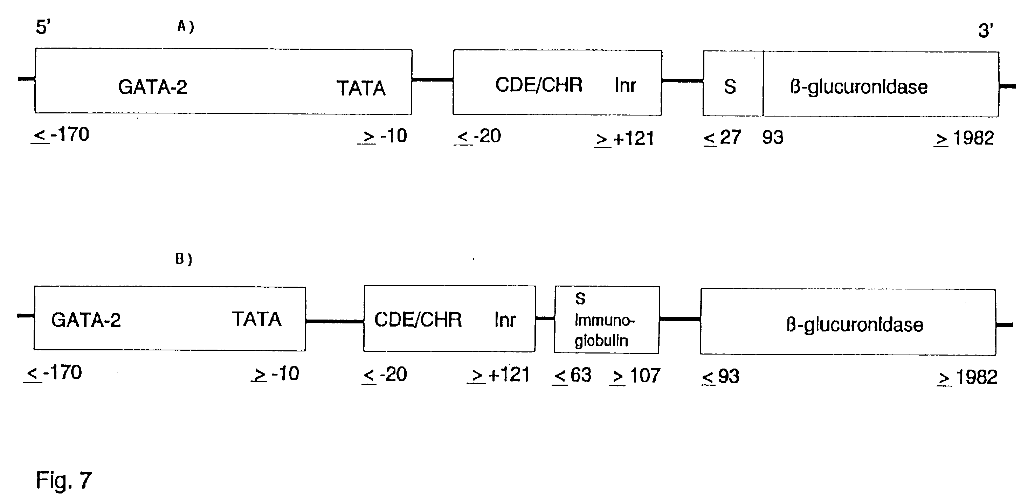

Die beschriebene chimäre Endothelin-1 Promotormodul-Transkriptionseinheit wird an ihren 3' Enden mit dem 5'-Terminus einer DNA, die den kompletten kodierenden Bereich der humanen β-Glucuronidase (Position ≤ 27 bis ≥ 1982; Oshima et al., PNAS USA 84, 685 (1987)), verknüpft. Diese DNA enthält auch die für eine Sekretion notwendige Signalsequenz (22 N-terminale Aminosäuren). Zur Erleichterung der zellulären Ausschleusung ist diese Signalsequenz bevorzugt auszutauschen gegen die Signalsequenz des Immunglobulins (Position ≤ 63 bis ≥ 107; Riechmann et al., Nature 332, 323 (1988), siehe Fig. 7). Transkriptionskontrolleinheiten und die DNA für β-Glucuronidase werden in pUC19/19 oder Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.

### 2.8. Wirksamkeit des Wirkstoffes

Ein Wirkstoff gemäß der vorliegenden Erfindung ermöglicht nach lokaler Applikation beispielsweise an den Ort des Tumors oder nach intrakranieller bzw. subarachnoidaler Gabe oder systemischer, bevorzugt intravenöser oder intraarterieller Gabe, daß durch die Kombination aus gewebespezifischer Aktivatorsequenz (UAS) und zellzyklusreguliertem Repressormodul vorwiegend, wenn nicht ausschließlich nur proliferierende Endothelzellen oder Nachbarzellen von diesen proliferierenden Endothelzellen Substanzen ausscheiden, die entweder selbst die Proliferation hemmen oder die Entstehung von antiproliferativ oder zytostatisch wirkenden Substanzen auslösen. Durch Hemmung der Endothelzellproliferation oder aber auch durch die Freisetzung von zytostatischen Substanzen kommt es zur Hemmung des Tumorwachstums. Da durch den erfindungsgemäßen Wirkstoff die Entstehung der antitumoralen Substanz auf den Ort des Tumorwachstums und der durch den Tumor verursachten Angiogenese beschränkt ist, ist die Verträglichkeit des Wirkstoffes gut.

Da der Wirkstoff sowohl durch seine Zell- als auch Zellzyklusspezifität ein hohes Maß an Sicherheit verspricht, kann er auch in hohen Dosierungen und, falls notwendig, mehrmals in Abständen von Tagen oder Wochen zur Therapie von Tumorerkrankungen angewandt werden.

Legende zu Fig. 1 - 7:
Fig. 1:
   Nukleotidsequenz des cdc25C - Promotorbereichs mit den *in vivo* gefundenen Proteinbindungsstellen (genomisches DMS Footprinting; (gefüllte Kreise): vollständiger konstitutiver Schutz; o (offene Kreise): partieller konstitutiver Schutz; * (Sternchen): zellzyklusregulierter, G1-spezifischer Schutz). CBS: constitutive binding site; CDE: cell cycle-dependent element. Grau unterlegte Bereiche zeigen die Y_{c}-Boxen (NF-Y Bindestellen) an. Startstellen sind durch gefüllte Quadrate markiert.
Fig. 2:
   Derepression des cdc25C-Promotors spezifisch in G₀ durch Mutation des cdc.
Fig. 3:
   Schematische Darstellung der cdc25C Enhancer-Regulation durch das CDE.
Fig. 4:
   G₀ / G₁ - spezifische Repression des SV40-Enhancers durch das CDE.Die Zahlen rechts bedeuten Induktion in G 2 (im Vergleich zur Kontrolle ≙ 1)
Fig. 5:
   Homologien im CDE-CHR-Bereich und den 5' gelegenen Yc-Boxen in den cdc25C, cyclin A und cdc2-Promotoren
Fig. 6:
   Chimäre Konstrukte bestehend aus verschiedenen Anteilen des humanen Endothelin-1 Promotors, dem 3' fusionierten Promotormodul mit den CDE und CHR Repressorelementen sowie einer DNA für humane β-glucuronidase (kompletter kodierender Bereich, Position ≤ 239 - ≥ 2194; Oshima et al., PNAS USA 84, 685 (1987) als Effector. Positionsangaben beziehen sich auf die Angaben von Wilson et al., Mol. Cell. Biol, 10, 4854 (1990) für das Endothelin-1-Gen bzw. auf das von Lucibello et al., EMBO J. 15 , 132 (1995) verwendete System für cdc25C.
Fig. 7:
   Positionsangabe der Signalsequenz (MGWSCIILFLVATAT) des Immunglobulins (HuVHCAMP) beziehen sich auf Riechmann et al., Nature 332. 323 (1988)
   Alternative: Einfügung des Signalpeptids des Ig zur besseren extrazellulären Ausschleusung der β-glucuronidase (Fig. 7B)

**Tabelle 1:**

| Rolle von CDE und CHR bei der zellzyklusregulierten Transkription von cdc25C, cyclin A und cdc2 | | | |
|---|---|---|---|
| ***Tab. 1*** | | | |
| | ***G***_{***0***} | ***Growing*** | ***Factor*** |
| ***wt*** | | | |
| cdc25C | 0.8 | 13.1 | 17.5 |
| cyclin A | 0.7 | 27.1 | 41.7 |
| cdc2 | 1.0 | 41.2 | 41.2 |
| | | | |

| ***mCDE(-13)*** | | | |
|---|---|---|---|
| cdc25C | 7.6 | 11.6 | 1.5 |
| cyclin A | 13.4 | 23.9 | 1.8 |
| cdc2 | 11.3 | 33.9 | 3.0 |
| | | | |

| ***mCHR(-6/-3)*** | | | |
|---|---|---|---|
| cdc25C | 14.4 | 21.0 | 1.5 |
| cyclin A | 15.5 | 28.3 | 1.8 |
| cdc2 | 18.6 | 38.6 | 2.1 |

Ergebnisse transienter Transfektionen in HIH3T3-Zellen sind als RLUs/1000 dargestellt. mCDE: mutiertes CDE (Pos. -13: G → T); mCHR: mutiertes CHR (Pos. -6 bis -3).

## Patentansprüche

1. Wirkstoff zur Prophylaxe oder Therapie von Tumorerkrankungen, dadurch charakterisiert, daß er ein DNA-Konstrukt enthält, welches aus einer Aktivatorsequenz, einem zellzyklusregulierten Promotormodul und einer DNA-Sequenz codierend für eine antitumorale Substanz besteht.

2. Wirkstoff nach Anspruch 1, bei welchem das Promotormodul die Elemente CDE-CHR-Inr besitzt und die Positionen ≤ -20 bis ≥ +30 des cdc25C Promotorbereiches enthält (Nukleotidsequenz GCTGGCGGAAGGTTTGAATGGTCAACGCCTGCGGCTGTTGATATTCTTG); Sequenz 2, wobei CDE das "cell cycle dependent element" (Nukleotidsequenz TGGCGG), Sequenz 3, CHR die "cell cycle gene homology region" (Nukleotidsequenz GTTTGAA) und Inr die Initiationsstelle (Position +1) sowie die für die Initiation wichtigen benachbarten Sequenzen darstellen.

3. Wirkstoff nach Anspruch 1 enthaltend eine Aktivatorsequenz (Promotor- oder Enhancersequenz), welche durch Transkriptionsfaktoren, die in Endothelzellen oder in Zellen in unmittelbarer Nachbarschaft zu proliferierenden Endothelzellen gebildet werden, reguliert wird.

4. Wirkstoff nach Anspruch 1 enthaltend:
- den CMV-Promotor, den CMV-Enhancer oder den SV40 Promotor oder
- die Promotorsequenz für den endothelialen Glucose-1-Transporter, Endoglin, VEGF-Rezeptor-1 oder -2, Rezeptortyrosinkinase til-1 oder til-2, B61 Rezeptor, B61 Ligand, Endothelin, insbesondere Endothelin-B, -1, Mannose-6-Phosphat-Rezeptor, IL-1α oder IL-1β, IL-1-Rezeptor, VCAM-1, von Willebrand Faktor oder
- eine synthetische Aktivatorsequenz aus oligomerisierten Bindestellen für Transkriptionsfaktoren, die preferentiell oder selektiv in Endothelzellen aktiv sind wie GATA-2, dessen Bindungsstelle 5'-TTATCT-3' ist oder
- die Promotorsequenz für VEGF oder die Enhancersequenz für VEGF oder die cDNA-Sequenz für c-SRC oder für v-SRC, welche das VEGF-Gen regulieren oder
- die Promotorsequenz des Maus-Mammatumor-Virus oder die Promotorsequenz eines Steroidrezeptors.

5. Wirkstoff nach Anspruch 1-4, bei welchem die DNA-Sequenz für den antitumoralen Wirkstoff
- das Retinoblastomprotein p1 10 oder p107 und p130 Proteine oder
- das p53 Protein ist oder
- das p21 Protein, p16 Protein oder einen anderen "Cyclin-dependent Kinase (cdK)"-Inhibitor oder
- das GADD45 Protein oder
- das bak Protein oder
- ein Protein, welches die Angiogenese inhibiert oder
- ein Protein, welches eine zytostatische Wirkung aufweist oder
- ein Protein, welches Entzündungen stimuliert oder
- ein Enzym für die Spaltung von Vorstufen von Zytostatika in Zytostatika kodiert.

6. Wirkstoff nach Anspruch 5,
- bei welchem das Retinoblastomprotein (pRb/p110) durch Austausch der Aminosäuren in den Positionen 246, 350, 601, 605, 780, 786, 787, 800 und
- 804 nicht mehr phosphorylierbar wird, ohne jedoch seine
- Bindungsaktivität mit dem großen T-Antigen einzubüßen, beispielsweise, daß die Aminosäuren Thr-246, Ser-601, Ser-605, Ser-780, Ser-786, Ser-787 und Ser-800 mit Ala, der Aminosäure Thr-350 mit Arg und der Aminosäure Ser-804 mit Glu ausgetauscht werden oder
- bei welchem das p107 Protein in analoger Weise wie das pRb/110 mutiert ist oder
- bei welchem das p130 Protein in analoger Weise wie das pRb/110 mutiert ist.

7. Wirkstoff nach Anspruch 5, bei welchem das Protein p53 C-terminal verkürzt ist um das Serin 392.

8. Wirkstoff nach Anspruch 1-4, bei welchem die antitumorale Substanz Plasminogenaktivatorinhibitor-1 (PAI-1), PAI-2, PAI-3, Angiostatin, Platelet Factor-4, TIMP-1, TIMP-2 oder TIMP-3 ist.

9. Wirkstoff nach Anspruch 1-4, bei welchem die antitumorale Substanz Perforin, Granzym, IL-2, IL-4, IL-12, Interferon wie IFNα, IFNβ, IFNγ, TNFα, TNFβ, Oncostatin M, RANTES, MCAF, IL-8, MIP-1α, MIP-1β, NAP-2, IL-3, IL-5, LIF, IL-11 oder IL-13 ist.

10. Wirkstoff nach Anspruch 9, bei welchem die antitumorale Substanz ein Fusionsprotein mit dem Fc-Fragment des Immunglobulins darstellt.

11. Wirkstoff nach Anspruch 1-4, bei welchem die antitumorale Substanz ein Enzym darstellt und dieses Enzym eine Herpes Simplex Virus Thymidin-Kinase, Cytosindeaminase, Varizella Zoster Virus Thymidin-Kinase, Nitroreduktase, β - Glucuronidase (im besonderen eine humane, pflanzliche oder bakterielle β-Glucuronidase), Carboxypeptidase (vorzugsweise von Pseudomonas), Lactamase (vorzugsweise von Bazillus cereus), Pyroglutamat-Aminopeptidase, D-Aminopeptidase, Oxidase, Peroxidase, Phosphatase, Hydroxynitrillyase, Protease, Esterase oder eine Glycosidase ist.

12. Wirkstoff nach Anspruch 11, bei welchem durch Punktmutationen der DNA-Sequenz des Enzyms die lysosomale Speicherung verringert und die extrazelluläre Ausschleusung erhöht ist.

13. Wirkstoff nach Anspruch 11, bei welchem die Signalsequenz des Enzyms ausgetauscht ist durch eine heterologe Signalsequenz zur Verbesserung der extrazellulären Ausschleusung.

14. Wirkstoff nach Anspruch 1-13, welcher die DNA-Sequenzen von mehreren gleichen oder unterschiedlichen antitumoralen Substanzen enthält, wobei jeweils zwei DNA-Sequenzen durch eine DNA-Sequenz für die internal ribosome entry site miteinander verbunden sind.

15. Wirkstoff nach Anspruch 1-13, eingefügt in einen Vektor.

16. Wirkstoff nach Anspruch 15, bei welchem der Vektor ein Virus ist.

17. Wirkstoff nach Anspruch 16, bei welchem das Virus ein Retrovirus, Adenovirus, Adeno-assoziiertes Virus, Herpes Simplex Virus oder Vaccinia Virus darstellt.

18. Wirkstoff nach Anspruch 1-14 eingefügt in ein Plasmid.

19. Wirkstoff nach Anspruch 15-18, zubereitet in einem kolloidalen Dispersionssystem.

20. Wirkstoff nach Anspruch 19, bei welchem das kolloidale Dispersionssystem Liposomen sind.

21. Wirkstoff nach Anspruch 20, bei welchem das kolloidale Dispersionsystem Polylysin-Liganden sind.

22. Wirkstoff nach Anspruch 15-21, ergänzt um einen Liganden, welcher an Membranstrukturen von Endothelzellen bindet.

23. Wirkstoff nach Anspruch 22, bei welchem der Ligand
- ein polyklonaler oder monoklonaler Antikörper oder ein Antikörperfragment hiervon ist, der mit seinen variablen Domänen an Membranstrukturen von Endothelzellen bindet,
- ein Zytokin oder Wachstumsfaktor oder ein Fragment bzw. eine Teilsequenz hiervon ist, der an Rezeptoren auf glatten Muskelzellen bindet-
- ein Adhäsionsmolekül darstellt, wie SLeX, LFA-1, MAC-1, LECAM-1 oder VLA-4.

24. Wirkstoff nach Anspruch 23, bei welchem die Membranstruktur von Endothelzellen ein Rezeptor darstellt für Mannose, IL-1 oder einen Wachstumsfaktor, wie PDGF, FGF, VEGF, TGFβ.

25. Wirkstoff nach Anspruch 1-24 in einer Arzneimittelzubereitung für die intravenöse, intraarterielle, intracavitäre Injektion, für die Injektion in Gewebe, in Gewebespalten oder zur lokalen Verabreichung.

## Claims

1. An active compound for the prophylaxis or therapy of tumor diseases, which comprises a DNA construct which is composed of an activator sequence, a cell cycle-regulated promoter module and a DNA sequence encoding an antitumoral substance.

2. An active compound as claimed in claim 1, in which the promoter module possesses the elements CDE-CHR-Inr and comprises positions # -20 to ∃ +30 of the cdc25C promoter region (nucleotide sequence GCTGGCGGAAGGTTTGAATGGTCAACGCCTGCGGCTGTTGATATTCTTG); sequence 2, with CDE representing the cell cycle-dependent element (nucleotide sequence TGGCGG), sequence 3, CHR representing the cell cycle gene homology region (nucleotide sequence GTTTGAA) and Inr representing the initiation site (position +1) and the neighboring sequences which are important for initiation.

3. An active compound as claimed in claim 1, which comprises an activator sequence (promoter sequence or enhancer sequence) which is regulated by transcription factors which are formed in endothelial cells or in cells which are in the immediate vicinity of proliferating endothelial cells.

4. An active compound as claimed in claim 1, which comprises
- the CMV promoter, the CMV enhancer or the SV40 promoter, or
- the promoter sequence for the endothelial glucose-1-transporter, endoglin, VEGF receptor 1 or 2, receptor tyrosine kinase til-1 or til-2, B61 receptor, B61 ligand, endothelin, in particular endothelin B or endothelin 1, mannose-6-phosphate receptor, IL-1α or IL-1β, IL-1 receptor, VCAM-1 or von Willebrand factor, or
- a synthetic activator sequence which is composed of oligomerized binding sites for transcription factors which are preferentially or selectively active in endothelial cells, such as GATA-2, whose binding side is 5'-TTATCT-3', or
- the promoter sequence for VEGF or the enhancer sequence for VEGF, or the cDNA sequence for c-SRC or for v-SRC, which regulate the VEGF gene, or
- the promoter sequence of mouse mammary tumor virus or the promoter sequence of a steroid receptor.

5. An active compound as claimed in claims 1 - 4, in which the DNA sequence for the antitumoral active compound encodes,
- retinoblastoma protein p110 or p107 and p130 proteins, or
- protein p53, or
- protein p21, protein p16 or another cyclindependent kinase (cdK) inhibitor, or
- GADD45 protein, or
- bak protein, or
- a protein which inhibits angiogenesis, or
- a protein which exhibits a cytostatic effect, or
- a protein which stimulates inflammations, or
- an enzyme for cleaving precursors of cytostatic agents thereby forming cytostatic agents.

6. An active compound as claimed in claim 5,
- in which the retinoblastoma protein (pRb/p110) is rendered no longer phosphorylatable by replacing the amino acids in positions 246, 350, 601, 605, 780, 786, 787, 800 and 804, without, however, forfeiting its binding activity with the large T antigen, for example with the amino acids Thr-246, Ser-601, Ser-605, Ser-780, Ser-786, Ser-787 and Ser-800 being replaced with Ala, the amino acid Thr-350 being replaced with Arg and the amino acid Ser-804 being replaced with Glu, or
- in which protein p107 is mutated in an analogous manner to pRb/110, or
- in which protein p130 is mutated in an analogous manner to pRb/110.

7. An active compound as claimed in claim 5, in which protein p53 is truncated C-terminally by removing serine 392.

8. An active compound as claimed in claims 1 - 4, in which the antitumoral substance is plasminogen activator inhibitor 1 (PAI-1), PA1-2, PAI-3, angiostatin, platelet factor 4, TIMP-1, TIMP-2 or TIMP-3.

9. An active compound as claimed in claims 1 - 4, in which the antitumoral substance is perforin, granzyme, IL-2, IL-4, IL-12, interferon, such as IFNα, IFNρ, IFNγ, TNFα or TNFβ, oncostatin M, RANTES, MCAF, IL-8, MIP-1α, MIA-1β, NAP-2, IL-3, IL-5, LIF, IL-11 or IL-13.

10. An active compound as claimed in claim 9, in which the antitumoral substance is a fusion protein comprising the Fc fragment of the immunoglobulin.

11. An active compound as claimed in claims 1 - 4, in which the antitumoral substance is an enzyme and this enzyme is a herpes simplex virus thymidine kinase, cytosine deaminase, varicella zoster virus thymidine kinase, nitroreductase, β-glucuronidase (in particular a human, plant or bacterial β-glucuronidase), carboxypeptidase (preferably from pseudomonas), lactamase (preferably from Bacillus cereus), pyroglutamate aminopeptidase, D-aminopeptidase, oxidase, peroxidase, phosphatase, hydroxynitrile lyase, protease, esterase or a glycosidase.

12. An active compound as claimed in claim 11, whose lysosomal storage is decreased and whose extracellular secretion is increased as a result of point mutations of the DNA sequence of the enzyme.

13. An active compound as claimed in claim 11, in which the signal sequence of the enzyme is replaced with a heterologous signal sequence in order to improve extracellular secretion.

14. An active compound as claimed in claims 1 - 13, which comprises the DNA sequences of several identical or different antitumoral substances, with in each case two DNA sequences being linked to each other by way of a DNA sequence for the internal ribosome entry site.

15. An active compound as claimed in claims 1 - 13, which is inserted into a vector.

16. An active compound as claimed in claim 15, in which the vector is a virus.

17. An active compound as claimed in claim 16, in which the virus is a retrovirus, adenovirus, adenoassociated virus, herpes simplex virus or vaccinia virus.

18. An active compound as claimed in claims 1 - 14, which is inserted into a plasmid.

19. An active compound as claimed in claims 15 - 18, which is prepared in a colloidal dispersion system.

20. An active compound as claimed in claim 19, in which liposomes are the colloidal dispersion system.

21. An active compound as claimed in claim 20, in which polylysine/ligands are the colloidal dispersion system.

22. An active compound as claimed in claims 15 - 21, which is supplemented with a ligand which binds to membrane structures of endothelial cells.

23. An active compound as claimed in claim 22, in which the ligand
- is a polyclonal or monoclonal antibody, or an antibody fragment thereof, which binds, by its variable domains, to membrane structures of endothelial cells,
- is a cytokine or growth factor, or a fragment or a constituent sequence thereof, which binds to receptors on smooth muscle cells, or
- is an adhesion molecule, such as SLeX, LFA-1, MAC-1, LECAM-1 or VLA-4.

24. An active compound as claimed in claim 23, in which the membrane structure of endothelial cells is a receptor for mannose, IL-1 or a growth factor, such as PDGF, FGF, VEGF or TGFβ.

25. An active compound as claimed in claims 1 - 24, in a pharmaceutical preparation for intravenous, intraarterial or intracavitary injection, of for injection into tissue or into tissue gaps, or for local administration.

## Revendications

1. Substance active pour la prophylaxie ou la thérapie de maladies tumorales, caractérisée en ce qu'elle contient un produit de construction de type ADN qui est constitué d'une séquence d'activateur, d'un module promoteur régulé par le cycle cellulaire et d'une séquence d'ADN codant pour une substance antitumorale.

2. Substance active selon la revendication 1, dans laquelle le module promoteur possède les éléments CDE-CHR-Inr et contient les positions ≤ -20 à ≥ +30 de la région de promoteur cdc25c (séquence nucléotidique GCTGGCGGAAGGTTTGAATGGTCAACGCCTGCGGCTGTTGATATTCTTG}; la séquence 2, CDE représentant l'élément dépendant du cycle cellulaire *(cell cycle dépendent element)* (séquence nucléotidique TGGCGG), la séquence 3, CHR représentant la région d'homologie de gène de cycle cellulaire *(cell cycle gene homology region)* (séquence nucléotidique GTTTGAA) et Inr représentant le site d'initiation (position +1), ainsi que les séquences voisines importantes pour l'initiation.

3. Substance active selon la revendication 1, contenant une séquence d'activateur (séquence de promoteur ou d'activateur) qui est régulée par des facteurs de transcription formés dans des cellules endothéliales ou dans des cellules au voisinage immédiat de cellules endothéliales proliférantes.

4. Substance active selon la revendication 1, contenant :
- le promoteur de CMV, l'activateur de CMV ou le promoteur de SV40, ou
- la séquence de promoteur pour le transporteur endothélial de glucose 1, pour l'endogline, pour le récepteur de VEGF 1 ou 2, la tyrosine kinase de récepteur til-1 ou til-2, le récepteur de B61, le ligand B61, l'endothéline, en particulier l'endothéline -B ou -1, le récepteur de mannose-6-phosphate, IL-1α ou IL-1β, le récepteur d'IL-1, VCAM-1, le facteur von Willebrand, ou
- une séquence d'activateur synthétique à base de sites de liaison oligomérisés pour des facteurs de transcription qui sont activés préférentiellement ou sélectivement dans des cellules endothéliales, tels que GATA-2, dont le site de liaison est 5'-TTATCT-3'.
- la séquence de promoteur pour VEGF ou la séquence d'activateur pour VEGF ou la séquence d'ADNc pour c-Src ou pour v-Src qui régulent le gène de VEGF, ou
- la séquence de promoteur du virus de tumeur mammaire murine ou la séquence de promoteur d'un récepteur stéroïdien.

5. Substance active selon les revendications 1 à 4, dans laquelle la séquence d'ADN code pour la substance active antitumorale qui est
- la protéine de rétinoblastome p110 ou p107 et la protéine p130 ou
- la protéine p53 ou
- la protéine p21, la protéine p16 ou un autre inhibiteur de "kinase dépendant de la cycline (cdK)" ou
- la protéine GADD45 ou
- la protéine bak ou
- une protéine qui inhibe l'angiogenèse ou
- une protéine qui a une action cytostatique ou
- une protéine qui stimule des inflammations ou
- une enzyme pour la coupure de précurseurs d'agents cytostatiques en agents cytostatiques.

6. Substance active selon la revendication 5
- dans laquelle la protéine de rétinoblastome (pRb/pl 10) n'est plus phosphorylable en raison d'un remplacement des aminoacides aux positions 246, 350, 601, 605, 780, 786, 787, 800 et 804, sans que toutefois ne soit perdue son activité de liaison au grand antigène T, par exemple, par remplacement des aminoacides Thr-246, Ser-601, Ser-605, Ser-780, Ser-786, Ser-787 et Ser-800 par Ala, de l'aminoacide Thr-350 par Arg et de l'aminoacide Ser-804 par Glu, ou
- dans laquelle la protéine p107 est mutée comme la protéine pRb/110 ou
- dans laquelle la protéine p130 est mutée comme la protéine pRb/110.

7. Substance active selon la revendication 5, dans laquelle la protéine p53 est raccourcie de la sérine 392 à l'extrémité C-terminale.

8. Substance active selon l'une des revendications 1 à 4, dans laquelle la substance antitumorale est l'inhibiteur d'activateur du plasminogène 1 (PAI-1), PAI-2, PAI-3, l'angiostatine, le facteur plaquettaire 4, TIMP-1, TIMP-2 ou TIMP-3.

9. Substance active selon l'une des revendications 1 à 4, dans laquelle la substance antitumorale est la perforine, la granzyme, IL-2, IL-4, IL-12, un interféron tel que IFNα, IFNβ, IFNγ, le TNFα, le TNFβ, l'oncostatine M, RANTES, MCAF, IL-8, MIP-1α, MIP-1β, NAP-2, IL-3, IL-5, LIF, IL-11 ou IL-13.

10. Substance active selon la revendication 9, dans laquelle la substance antitumorale représente une protéine de fusion avec le fragment Fc de l'immunoglobuline.

11. Substance active selon l'une des revendications 1 à 4, dans laquelle la substance antitumorale représente une enzyme et cette enzyme est la thymidine kinase d'herpès simplex virus, la cytosine désaminase, la thymidine kinase du virus varicelle-zona, la nitroréductase, une β-glucuronidase (en particulier une β-glucuronidase humaine, végétale ou bactérienne), une carboxypeptidase (de préférence de *Pseudomonas),* une lactamase (de préférence de *Bacillus cereus*), la pyroglutamate aminopeptidase, une D-aminopeptidase, oxydase, peroxydase, phosphatase, hydroxynitrilase, protéase, estérase ou une glycosidase.

12. Substance active selon la revendication 11, dans laquelle le stockage lysosomique est diminué et l'excrétion extracellulaire est accrue par des mutations ponctuelles de la séquence d'ADN de l'enzyme.

13. Substance active selon la revendication 11, dans laquelle la séquence signal de l'enzyme est remplacée par une séquence signal hétérologue, pour l'amélioration de l'excrétion extracellulaire.

14. Substance active selon l'une des revendications 1 à 13, qui contient les séquences d'ADN de plusieurs substances antitumorales identiques ou différentes, deux séquences d'ADN étant dans chaque cas reliées l'une à l'autre par une séquence d'ADN codant pour le site d'entrée interne du ribosome.

15. Substance active selon l'une des revendications 1 à 13, insérée dans un vecteur.

16. Substance active selon la revendication 15, dans laquelle le vecteur est un virus.

17. Substance active selon la revendication 16, dans laquelle le virus représente un rétrovirus, un adénovirus, un virus associé à un adénovirus, l'herpès simplex virus ou le virus de la vaccine.

18. Substance active selon l'une des revendications 1 à 14, insérée dans un plasmide.

19. Substance active selon l'une des revendications 15 à 18, préparée dans un système de dispersion colloïdale.

20. Substance active selon la revendication 19, dans laquelle le système de dispersion colloïdale consiste en des liposomes.

21. Substance active selon la revendication 20, dans laquelle le système de dispersion colloïdale consiste en des ligands-polylysine.

22. Substance active selon l'une des revendications 15 à 21, complétée par un ligand qui se lie à des structures membranaires de cellules endothéliales.

23. Substance active selon la revendication 22, dans laquelle le ligand
- est un anticorps monoclonal ou polyclonal ou un fragment d'anticorps de celui-ci, qui se lie par ses domaines variables à des structures membranaires de cellules endothéliales,
- est une cytokine ou un facteur de croissance, ou un fragment ou une séquence partielle de ceux-ci, qui se lie à des récepteurs sur des cellules musculaires lisses,
- représente une molécule d'adhérence, telle que SLeX, LFA-1, MAC-1, LECAM-1 ou VLA-4.

24. Substance active selon la revendication 23, dans laquelle la structure membranaire de cellules endothéliales représente un récepteur pour le mannose, IL-1 ou un facteur de croissance, tel que PDGF, FGF, VEGF, TGFβ.

25. Substance active selon l'une des revendications 1 à 24, dans une composition de médicament pour l'injection intraveineuse, intraartérielle, intracavitaire, pour l'injection dans un tissu, dans des interstices tissulaires ou pour l'administration locale.
